# EUROPÄISCHE PATENTSCHRIFT

(11) **EP 1 459 678 B1**
(45) Veröffentlichungstag und Bekanntmachung des Hinweises auf die Patenterteilung: **30.08.2006**
(21) Anmeldenummer: 04004767.2
(22) Anmeldetag: 02.03.2004
(51) Int. Cl.: A61B 5/00, G06F 15/02, G06F 19/00

(54) **Vorrichtung zur Bestimmung wenigstens eines ernährungsspezifischen Wertes**
Apparatus for determining at least one value specific to nutrition
Dispositif pour déterminer au moins une valeur spécifique à la nutrition

(30) Priorität: 21.03.2003 DE 10312746; 16.09.2003 DE 10342650
(43) Veröffentlichungstag der Anmeldung: 22.09.2004
(73) Patentinhaber: Fresenius Kabi Deutschland GmbH, 61346 Bad Homburg v.d.H. (DE)
(72) Erfinder: Reiss, Christiane, Dr., 65824 Schwalbach (DE)
(74) Vertreter: Knefel, Cordula

(56) Entgegenhaltungen:
- US-A- 5 717 938
- US-A1- 2002 133 378
- INET, [Online] 23. Februar 2003 (2003-02-23), XP002288263 Gefunden im Internet: URL:http://www.iwa.de/2-d/produkte/produkt -template.asp?Gruppe=1&from=site> [gefunden am 2004-07-13] -& INET, [Online] 23. Februar 2003 (2003-02-23), XP002288561 Gefunden im Internet: URL:http://web.archive.org/web/20030223203 017/http://www.iwa.de/2-d/produkte/produkt -template.asp?Gruppe=1&from=site> [gefunden am 2004-07-13]

## Beschreibung

Die Erfindung betrifft eine Vorrichtung zur Bestimmung wenigstens eines ernährungsspezifischen Wertes.

Unter ernährungsspezifischen Werten im Sinne der Erfindung werden Werte verstanden, die eine reine Nahrungsaufnahme, eine Flüssigkeitsaufnahme, eine Aufnahme von Spurenelementen, von Mineralstoffen, Vitaminen, Eiweißen, Fetten, Kohlehydraten oder dergleichen betreffen.

Zur Bestimmung des Ernährungsstatus eines Patienten bedarf es in der Regel einer umfangreichen ärztlichen Untersuchung mit Laboranalysen.

Zum Stand der Technik (US 2002/0133378 A1) gehört ein Computer, mit dem eine Gewichtsabnahme oder Gewichtszunahme über einen bestimmten Zeitraum kontrolliert werden kann.

Der Computer unterstützt eine Person in einem Gewichtskontrollprogramm, welches über einen bestimmen Zeitraum ausgedehnt wird. Dieser Zeitraum wird in vorbestimmte Zeitintervalle unterteilt, und in diesen Intervallen wird die gesamte Kalorienzufuhr bestimmt. Unter anderem wird mit dem Computer der Bodymass-Index (BMI) bestimmt.

Diese zum Stand der Technik gehörende Vorrichtung hat den Nachteil, dass diese Vorrichtung relativ aufwändig ist, da sämtliche bestimmbaren Daten über eine Software zu ermitteln sind. Diese Software muss für die Vorrichtung erstellt werden. Darüber hinaus ist die Vorrichtung relativ aufwändig in der Bedienstruktur, so dass sich ein Nutzer erst einmal mit dieser Vorrichtung vertraut machen und einarbeiten muss.

Darüber hinaus ist mit dieser Vorrichtung eine exakte Bestimmung des Ernährungsstatus eines Patienten nicht möglich, da es hierfür zum Beispiel erforderlich ist, den prozentualen Gewichtsverlust, das heißt die Relation des aktuellen Körpergewichtes und des üblichen Körpergewichtes mitzubestimmen und zu berücksichtigen.

Weiterhin gehört zum Stand der Technik (INET, [Online] 23. Februar 2003 (2003-02-23), XP002288263 Gefunden im Internet: URL:http://www.iwa.de/2-d/produkte/produkt-template.asp?Gruppe=1&from=site> [gefunden am 2004-07-13]) eine Bodymass-Index-Scheibe zur Bewertung des Körpergewichtes eines Patienten. Mit dieser zum Stand der Technik gehörenden Scheibe kann ausgehend von Körpergröße und Gewicht des Patienten der Bodymass-Index eines Patienten bestimmt werden.

Diese zum Stand der Technik gehörende Vorrichtung hat den Nachteil, dass der Ernährungsstatus eines Patienten nicht bestimmt werden kann, da hier zum Beispiel noch ein prozentualer Gewichtsverlust mit eingeht. Diese zum Stand der Technik gehörende Scheibe reicht nicht für die Bestimmung eines Ernährungsstatus eines Patienten aus.

Weiterhin gehört zum Stand der Technik (US 5,717,938 A) ein elektronisches Buch. Dieses elektronische Buch zeichnet sich unter anderem dadurch aus, dass Datenträger mit verschiedenen Büchern eingelesen werden können. Diese Vorrichtung weist eine Reihe von Funktionstasten auf, die, je nach eingelesenem Buch, zur besseren Unterscheidung farbig gekennzeichnet sind.

Diese zum Stand der Technik gehörende Vorrichtung bestimmt keine patientenspezifischen Daten und wertet diese auch nicht aus.

Der Erfindung liegt das technische Problem zugrunde, eine Vorrichtung zur Bestimmung wenigstens eines ernährungsspezifischen Wertes zur Bestimmung eines Ernährungsstatus eines Patienten bereitzustellen, die einfach und schnell ohne Laboruntersuchung eine Information über den Ernährungsstatus des Patienten liefert.

Die Lösung dieses technischen Problems ist gekennzeichnet durch eine Vorrichtung zur Bestimmung wenigstens eines ernährungsspezifischen Wertes zur Bestimmung eines Ernährungsstatus eines Patienten mit einer Zuordnungseinheit zur Ermittlung wenigstens eines Zwischenwertes, wobei mittels der Zuordnungseinheit die Körpergröße und das Körpergewicht eines Patienten zur Ermittlung des Bodymass-Index (BMI) als ein Zwischenwert in Relation zueinander setzbar sind, und wobei mittels der Zuordnungseinheit (1) zusätzlich das aktuelle Körpergewicht und das übliche Körpergewicht eines Patienten zur Ermittlung des prozentualen Gewichtsverlustes als ein weiterer Zwischenwert in Relation setzbar sind, einem Informationsblock zur Ermittlung wenigstens eines weiteren Zwischenwertes sowie mit einer Auswerteeinheit zur Bestimmung eines Endwertes aus dem wenigstens einen Zwischenwert der Zuordnungseinheit und dem wenigstens einen Zwischenwert des Informationsblockeswobei die Zuordnungseinheit mindestens zwei relativ zueinander verstellbare Bauteile zur Ermittlung des Bodymass-Index aufweist, wobei die Zuordnungseinheit zur Ermittlung von zwei Zwischenwerten ausgebildet ist, wobei die Vorderseite zur Ermittlung des einen Zwischenwertes und die Rückseite zur Ermittlung des anderen Zwischenwertes ausgebildet ist, wobei die Zuordnungseinheit auf der Vorderseite eine Skala mit den Zwischenwerten zugeordneten Farben, Symbolen oder Zahlen und auf der Rückseite eine Skala mit den Zwischenwerten zugeordneten Farben, Symbolen oder Zahlen aufweist, und wobei die Auswerteeinheit Tastenfelder aufweist, die den Zwischenwerten zugeordneten Farben, Symbolen oder Zahlen entsprechen.

Mit der erfindungsgemäßen Vorrichtung können auf einfache Art und Weise mehrere patientenspezifische Werte bestimmt werden, die miteinander verknüpft einen Endwert ergeben, dem zu entnehmen ist, ob die Notwendigkeit besteht, den Patienten enteral oder parenteral zu ernähren.

Um die Auswertung der ermittelten Zwischenwerte zur Bestimmung des gesuchten ernährungsspezifischen Endwertes zu erleichtern, wird gemäß der Erfindung vorgeschlagen, dass jedem ermittelten Zwischenwert über eine zugehörige Farbskala eine individuelle Farbe zuordbar ist. Diese jeweiligen, einem zuvor ermittelten Zwischenwert entsprechenden Farben sind dann zur Bestimmung des Endwertes in die vorteilhafterweise in der Art eines elektronischen Taschenrechners ausgebildete Auswerteeinheit eingebbar, wobei in der Auswerteeinheit mittels einer elektronisch, elektrisch, elektromechanisch oder mechanisch arbeitenden Datenverarbeitungseinheit aus den Zwischenwerten der Endwert bestimmt wird.

Alternativ zu der Zuordnung einer Farbe zu jedem ermittelten Zwischenwert ist es selbstverständlich auch möglich, den Zwischenwerten andere Unterscheidungsmerkmale, wie beispielsweise Symbole oder feste Zahlenwerte zuzuordnen.

Gemäß einer bevorzugten Ausführungsform der Erfindung sind mit der Zuordnungseinheit wenigstens zwei patientenspezifische Werte zur Ermittlung wenigstens eines zwischenwertes in Relation zueinander setzbar.

Gemäß einer bevorzugten Ausführungsform der Erfindung sind mit der Zuordnungseinheit wenigstens zwei patientenspezifische Werte zur Ermittlung wenigstens eines Zwischenwertes in Relation zueinander setzbar.

Gemäß einer praktischen Ausführungsform der Erfindung ist die Zuordnungseinheit aus wenigstens zwei relativ zueinander um eine gemeinsame Achse verdrehbaren Bauteilen bestehend aufgebaut, wobei beide Bauteile mit wenigstens einer Skala versehen sind. Dieser Aufbau der Zuordnungseinheit als Drehscheibe stellt eine besonders einfach und übersichtlich zu handhabende Ausgestaltungsform dar.

Alternativ ist es erfindungsgemäß möglich, die Zuordnungseinheit aus wenigstens zwei relativ zueinander verschiebbaren Bauteilen bestehend aufzubauen, wobei beide Bauteile mit wenigstens einer Skala versehen sind.

Zur Ermittlung wenigstens eines weiteren Zwischenwertes wird erfindungsgemäß vorgeschlagen, dass der Informationsblock vorgegebene allgemeine Angaben zum Gesundheitsstatus enthält, die mit den konkreten Angaben zum Gesundheitsstatus eines Patienten vergleichbar sind. Um einen besonders aussagekräftigen Endwert ermitteln zu können, sind die im Informationsblock enthaltenen, vorgegebenen allgemeinen Angaben zum Gesundheitsstatus beispielsweise in die Teilbereiche Nahrungsaufnahme, Schwere der Erkrankung und Alter des Patienten unterteilbar, so dass aus diesen Teilbereichen jeweils ein Zwischenwert ermittelbar ist.

Je mehr patientenspezifische Zwischenwerte zur Bestimmung des Endwertes herangezogen werden können, desto aussagekräftiger ist dieser ermittelte Endwert.

Die Datenverarbeitungseinheit der Auswerteeinheit arbeitet vorteilhafterweise mittels eines Auswertungsalgorithmus, der jeder unterschiedlichen Farbe eines Zwischenwertes einen unterschiedlichen Zahlenwert zuordnet. Der Endwert kann anschließend leicht erkennbar wiederum als optisches, insbesondere farbiges Signal ausgegeben werden.

Weiterhin wird mit der Erfindung vorgeschlagen, dass die Zuordnungseinheit, der Informationsblock sowie die Auswerteeinheit auf einem faltbaren Grundkörper angeordnet sind, so dass die gesamte Vorrichtung auf eine handliche Größe zusammenlegbar ist.

Zusätzlich kann die Vorrichtung einen auf dem Grundkörper angeordneten Frageblock umfassen.

Schließlich wird mit der Erfindung vorgeschlagen, dass am Grundkörper wenigstens eine Tasche ausgebildet ist, die insbesondere zur Aufbewahrung kartenförmiger Materialien, die beispielsweise auch als Trägermaterialien für zum Beispiel Aufkleber dienen können, geeignet ist. Diese Aufkleber oder Markierungselemente dienen zur von außen sichtbaren Kennzeichnung von Patientenkarteien. Die Aufkleber können beispielsweise als Farbpunkte ausgebildet sein, wobei die Farbpunkte farblich den ermittelten Werten entsprechen. Auf diese Art und Weise können die Patientenkarteien sehr leicht und übersichtlich markiert werden.

Weitere Merkmale und Vorteile der Erfindung ergeben sich anhand der nachfolgenden Beschreibung der zugehörigen Zeichnungen, in denen ein Ausführungsbeispiel einer erfindungsgemäßen Vorrichtung zur Bestimmung wenigstens eines ernährungsspezifischen Wertes dargestellt ist, und zwar zeigen:
- Fig. 1: eine Gesamtansicht einer erfindungsgemäßen Vorrichtung im aufgeklappten Zustand;
- Fig. 2: eine vergrößerte Ansicht der Zuordnungseinheit der Vorrichtung gemäß Fig. 1;
- Fig. 3: eine Rückansicht der Zuordnungseinheit gemäß Fig. 2;
- Fig. 4: eine vergrößerte Ansicht des Informationsblockes der Vorrichtung gemäß Fig. 1;
- Fig. 5: eine vergrößerte Ansicht der Auswerteeinheit der Vorrichtung gemäß Fig. 1;
- Fig. 6: eine Ansicht eines Frageblockes einer erfindungsgemäßen Vorrichtung;

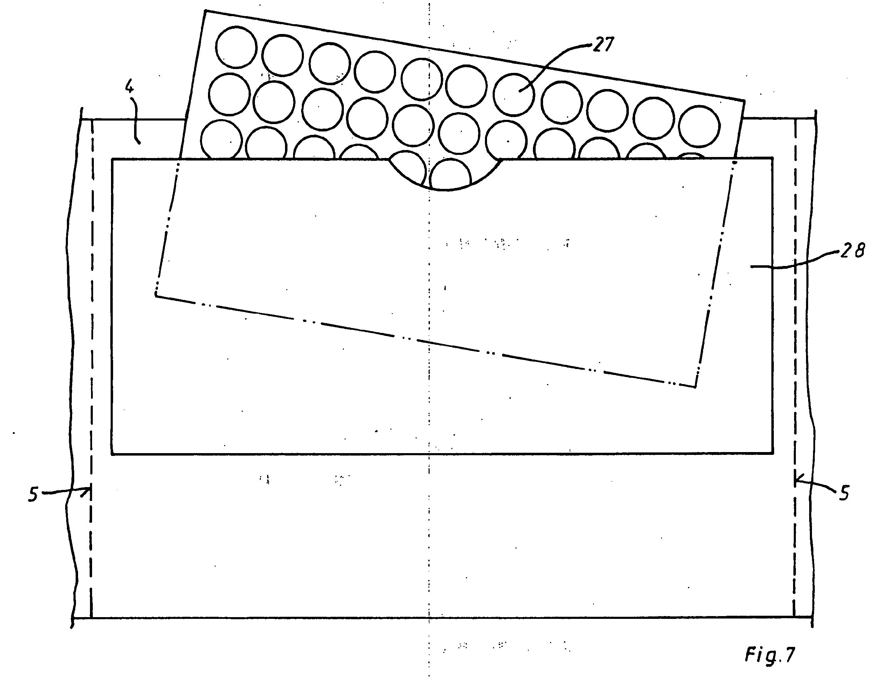

- Fig. 7: einen vergrößerten rückseitigen Ausschnitt der Vorrichtung gemäß Fig. 1.

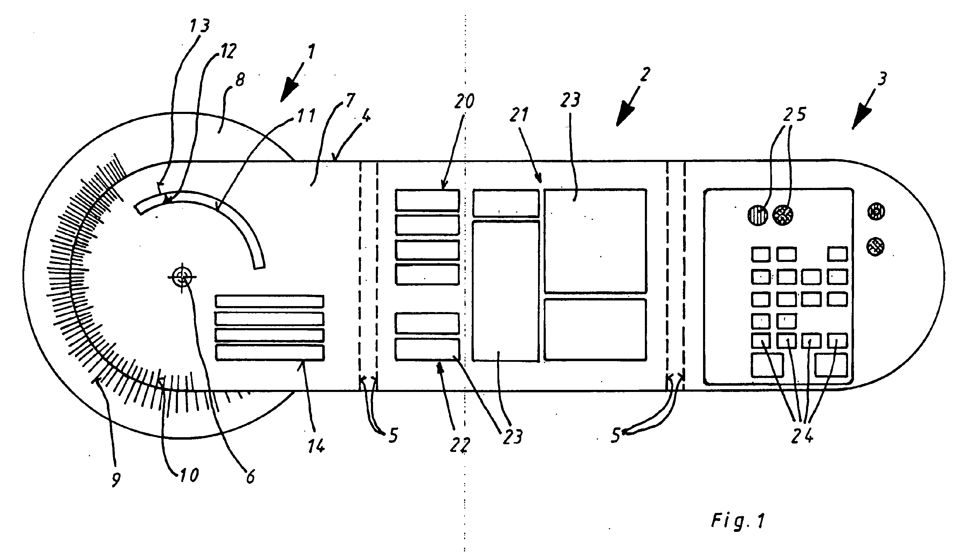

Fig. 1 zeigt eine Vorrichtung zur Bestimmung wenigstens eines ernährungsspezifischen Wertes im aufgeklappten Zustand. Die dargestellte Vorrichtung besteht im Wesentlichen aus einer Zuordnungseinheit (1), einem Informationsblock (2) sowie einer Auswerteeinheit (3). Die einzelnen Baugruppen (1, 2, 3) sind auf einem faltbaren Grundkörper (4) angeordnet, der vorteilhafterweise über Falze,(5) faltbar ist, so dass die gesamte Vorrichtung auf eine handliche Größe zusammenklappbar ist.

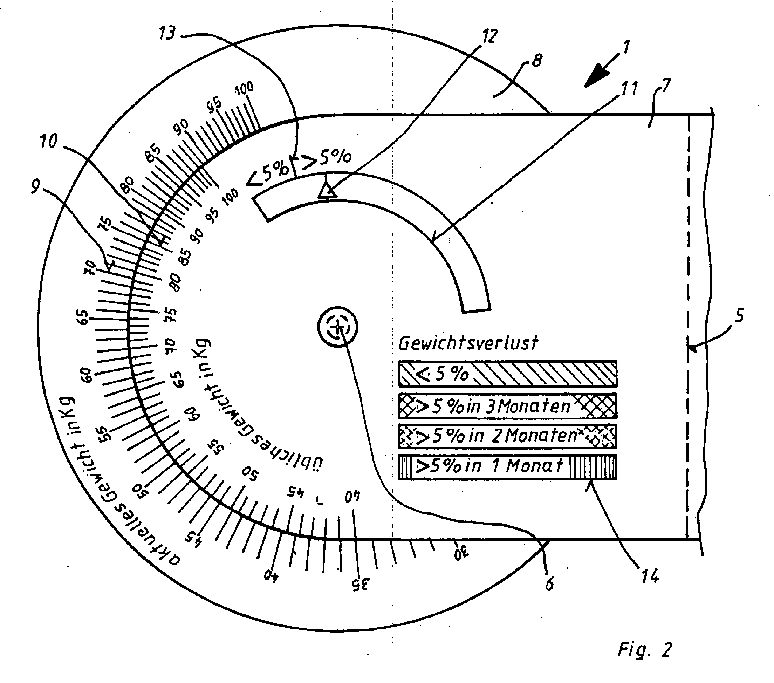

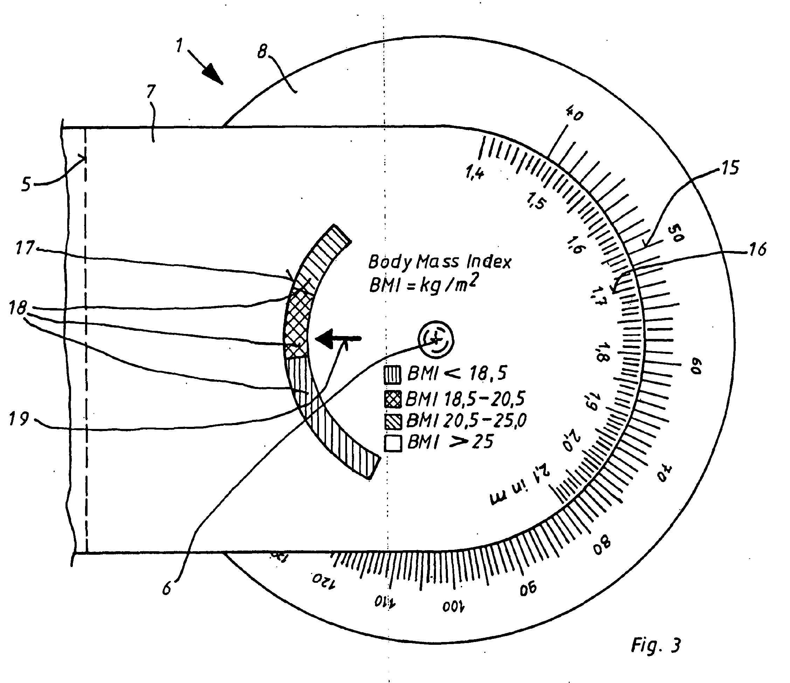

Fig. 2 und 3 zeigen vergrößerte Ansichten der Zuordnungseinheit (1), die beim dargestellten Ausführungsbeispiel aus zwei relativ zueinander um eine gemeinsame Achse (6) verdrehbaren Bauteilen, nämlich einem Halter (7) und einer drehbar in dem Halter (7) angeordneten Scheibe (8), besteht.

Zur Ermittlung eines prozentualen Gewichtsverlustes als einen ersten Zwischenwert weist bei den Darstellungen der Zuordnungseinheit (1) gemäß Fig. 1 und 2 die Scheibe (8) eine Skala (9) für das aktuelle Gewicht, das der Patient beispielsweise nach längerer Krankheit aufweist, und der Halter (7) eine Skala (10) für das übliche Gewicht des Patienten auf. Diese Gewichte werden durch Drehen der Scheibe (8) in dem Halter (7) in Relation zueinander gesetzt. Im vorliegenden Fall ist ein Normalgewicht von 69 Kilogramm zu einem aktuellen Gewicht von 61 Kilogramm in Relation gesetzt.

Das Ergebnis wird durch einen auf der Scheibe (8) angeordneten und durch eine Ausnehmung (11) im Halter (7) sichtbaren Pfeil (12) angezeigt, wozu die Ausnehmung (11) mit einer Markierung (13) versehen ist, die unterteilt, ob der ermittelte Gewichtsverlust oberhalb oder unterhalb eines vorgegebenen Prozentsatzes, im vorliegenden Fall 5 %, liegt. Über eine ebenfalls auf dem Halter (7) angeordnete Farbskala (14) wird dem über den Pfeil (12) angezeigten Ergebnis in Abhängigkeit der Höhe des ermittelten Gewichtsverlustes, kleiner oder größer 5 %, und in Abhängigkeit von der Geschwindigkeit des über 5 % betragenden Gewichtsverlustes eine entsprechende Farbe zugeordnet, die den ersten Zwischenwert darstellt.

Beim dargestellten Ausführungsbeispiel sind die den Zwischenwert verkörpernden Farben wie folgt zugeordnet: Grün für einen Gewichtsverlust kleiner 5 %; Gelb für einen Gewichtsverlust größer 5 % in drei Monaten; Orange für einen Gewichtsverlust größer 5 % in zwei Monaten und Rot für einen Gewichtsverlust größer 5 % in einem Monat.

Die Rückseite der in Fig. 2 dargestellten Zuordnungseinheit (1) ist in Fig. 3 dargestellt. Zur Ermittlung des Bodymass-Index (BMI), der Körpermassezahl, als einen weiteren Zwischenwert weist die Scheibe (8) eine Gewichtsskala (15) für das aktuelle Gewicht des Patienten und der Halter (7) eine Größenskala (16) für die Körpergröße des Patienten auf.

Durch Drehen der Scheibe (8) im Halter (7) wird das Gewicht des Patienten zur Körpergröße des Patienten in Relation gesetzt. Das Ergebnis wird durch eine auf der Scheibe (8) angeordnete und durch eine im Halter (7) ausgebildete Ausnehmung (17) sichtbare Farbskala (18) angezeigt. Die Ablesestelle ist durch einen Pfeil (19) gekennzeichnet.

Beim dargestellten Ausführungsbeispiel sind die den Zwischenwert verkörpernden Farben wie folgt zugeordnet: Rot für einen BMI-Wert kleiner 18,5; Gelb für einen BMI-Wert zwischen 18,5 und 20,5; Grün für einen BMI-Wert zwischen 20,5 und 25,0 und Weiß für einen für die Auswertung nicht interessanten BMI-Wert größer 25.

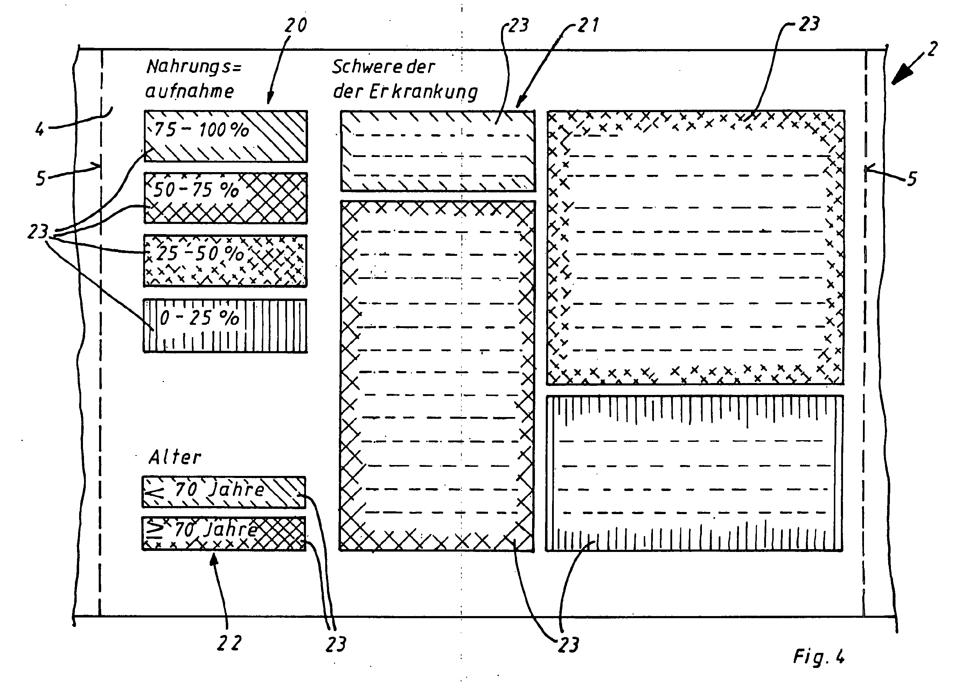

Mittels des in Fig. 4 dargestellten Informationsblockes (2) sind drei weitere Zwischenwerte ermittelbar. Der Informationsblock (2) enthält vorgegebene allgemeine Angaben zum Gesundheitsstatus, die zur Ermittlung weiterer Zwischenwerte mit den konkreten Angaben zum Gesundheitsstatus des Patienten verglichen werden. Bei der dargestellten Ausführungsform sind diese allgemeinen Angaben zum Gesundheitsstatus in die Teilbereiche Nahrungsaufnahme (Food Intake) (20), Schwere der Erkrankung (Severity of Diseases) (21) und Alter (Age) (22) des Patienten unterteilt, aus denen jeweils ein Zwischenwert ermittelbar ist, wozu die im Informationsblock (2) aufgeführten drei Teilbereiche in farbig abgestuften Informationsfeldern (23) angeordnet sind. Das aus jedem Teilbereich ermittelte farbige Informationsfeld (23) entspricht einem Zwischenwert.

Bei dem in Fig. 4 dargestellten Ausführungsbeispiel sind die die jeweiligen Zwischenwerte der Teilbereiche (20, 21, 22) verkörpernden Farben wie folgt zugeordnet:

Im Teilbereich (20) "Nahrungsaufnahme", in dem eine Unterteilung vorgenommen ist bezüglich der Nahrungsmenge, die der Patient aktuell aufnimmt, prozentual in Bezug gesetzt zu der Nahrungsmenge, die der Patient normalerweise zu sich nimmt: Grün für 75-100 %; Gelb für 50-75 %; Orange für 25-50 % und Rot für 0-25 %.

Im Teilbereich (21) "Schwere der Erkrankung": Grün zum Beispiel für "keine Stoffwechselerkrankung"; Gelb zum Beispiel für "Oberschenkelfraktur oder chronische Erkrankungen (beispielsweise Diabetes)"; Orange zum Beispiel für "Schlaganfall, Darmverschluss oder wiederholte Operationen" und Rot zum Beispiel für "Kopfverletzungen, Knochenmarktransplantationen oder Intensivpflegepatienten".

Im Teilbereich (22) "Alter": Grün für jünger als 70 Jahre und Gelb für 70 Jahre oder älter.

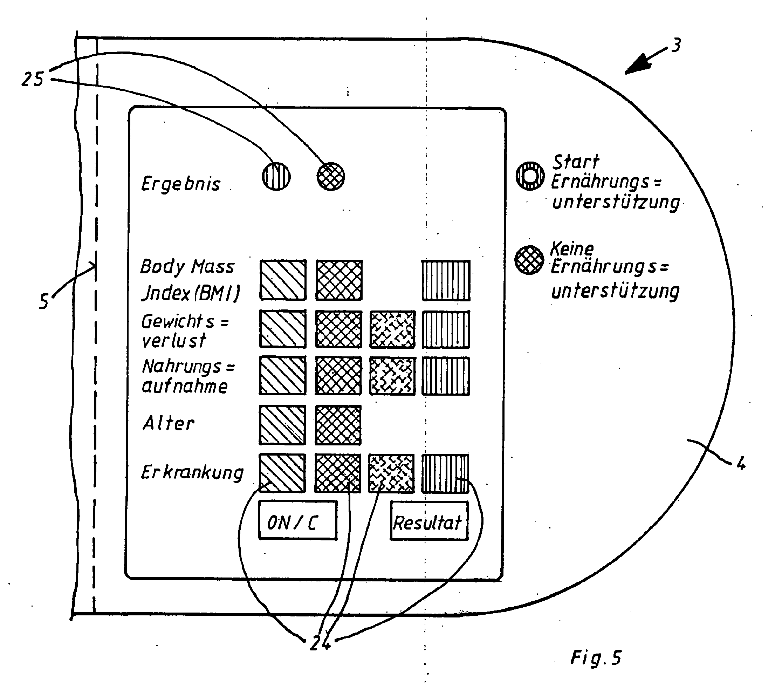

Das Bestimmen des ernährungsspezifischen Endwertes aus den mittels der Zuordnungseinheit (1) und dem Informationsblock (2) ermittelten Zwischenwerten erfolgt mittels der Auswerteeinheit (3) gemäß Fig. 5.

Die in der Art eines elektronischen Taschenrechners aufgebaute Auswerteeinheit (3) weist eine Mehrzahl farbiger Tastenfelder (24) auf, deren Farben den auswerterelevanten Farben der zuvor ermittelten Zwischenwerte entsprechen. Im vorliegenden Fall sind die Tastenfelder (24) in fünf Zeilen untereinander angeordnet, wobei jede Zeile einem der ermittelten Zwischenwerte entspricht, nämlich dem "Bodymass-Index (BMI)", dem "Gewichtsverlust", der "Nahrungsaufnahme", dem "Alter" sowie der "Schwere der Erkrankung".

Zur Bestimmung des Endwertes werden die den einzelnen Zwischenwerten entsprechenden Farben über die entsprechend farbig gekennzeichneten Tastenfelder (24) der den jeweiligen Zwischenwerten entsprechenden Zeilen in die Auswerteeinheit (3) eingegeben, die aus diesen Eingaben mittels eines nachfolgend näher beschriebenen Auswertungsalgorithmus den gewünschten ernährungsspezifischen Endwert berechnet.

Das den Endwert darstellende Ergebnis wird als farbiges optisches Signal in Ergebnisfeldern (25) angezeigt. Bei der dargestellten Ausführungsform sind die den Endwert verkörpernden Farben der Ergebnisfelder (25) wie folgt zugeordnet: Gelb für das Ergebnis, dass keine Ernährungsunterstützung notwendig ist und Rot für das Ergebnis, dass eine Ernährungsunterstützung (möglichst täglich) notwendig ist.

Die Auswerteeinheit (3) ist vorteilhaft als mit Solarzellen ausgestattete Solarrecheneinheit ausgebildet. Es ist jedoch auch möglich die Auswerteeinheit (3) batteriebetrieben oder in Kombination batterie- und solarbetrieben auszugestalten.

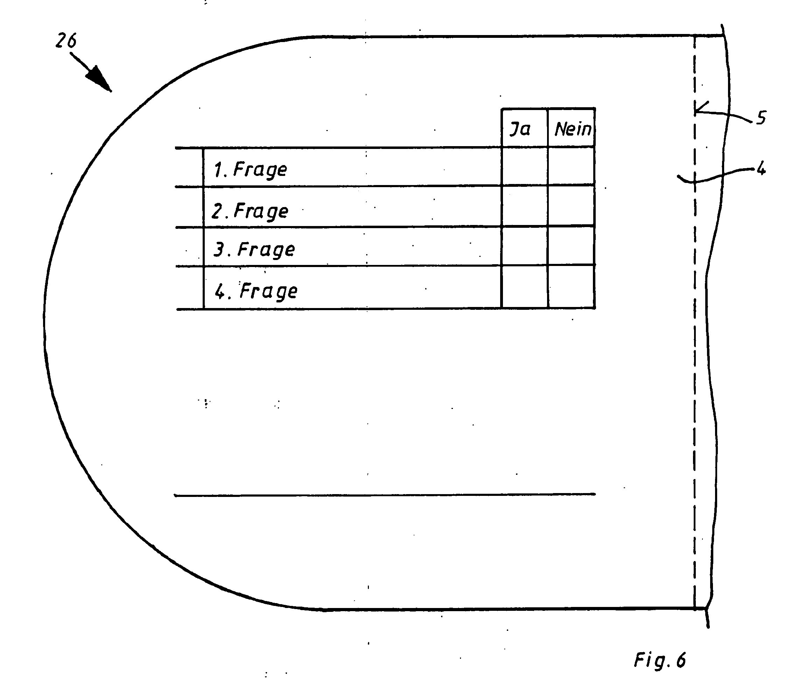

Zusätzlich zu den zuvor beschriebenen, zur Ermittlung der Zwischenwerte und des Endwertes notwendigen Baueinheiten (1, 2, 3) weist die dargestellte Vorrichtung noch einen in Fig. 6 dargestellten Frageblock (26) auf, der bei der vorliegenden Ausführungsform der Vorrichtung auf der Rückseite der Auswerteeinheit (3) gemäß Fig. 5 auf dem Grundkörper (4) der Vorrichtung angeordnet ist.

Mittels dieses Frageblockes (26), der mehrere nur mit "ja" oder "nein" zu beantwortende Fragen enthält, kann im Vorfeld einfach und schnell abgeklärt werden, ob der Gesundheitsstatus eines Patienten so ist, dass er eventuell einer Ernährungsunterstützung bedarf und somit mittels der zuvor beschriebenen Vorrichtung der ernährungsspezifische Endwert ermittelt werden sollte.

Der Einfachheit und Übersichtlichkeit halber sollten die Eingangsfragen des Frageblockes (26) so aufgebaut sein, dass deren Auswertung keinen Interpretationsspielraum bietet. Dies kann beispielsweise dadurch erreicht werden, dass die Endwertbestimmung mittels der Vorrichtung durchzuführen ist, sobald nur eine Frage mit "ja" beantwortet wird.

Zur Aufnahme kartenfömiger Materialien (27), wie beispielsweise Erläuterungen zur Bedienung der Vorrichtung, weist die Vorrichtung weiterhin eine als Einsteckfach ausgebildete Tasche (28) auf, die auf der Rückseite des Informationsblockes (2) am Grundkörper (4) der Vorrichtung angeordnet ist.

Die kartenförmigen Materialien (27) können auch als Trägermaterialien für Aufkleber dienen. Die Aufkleber wiederum sind zur von außen sichtbaren Kennzeichnung von Patientenkarteien, beispielsweise als Farbpunkte ausgebildet.

Die Bestimmung eines ernährungsspezifischen Wertes mittels der Vorrichtung geschieht wie folgt:

Zunächst wird mittels der Fragen des Frageblockes (26) abgeprüft, ob der zu untersuchende Patient ein potentieller Patient für eine Ernährungsunterstützung ist.

Ergibt diese Befragung, dass der Patient beispielsweise wegen eines hohen Gewichtsverlustes in den letzten Monaten oder einer schweren Erkrankung einer Ernährungsunterstützung bedarf, werden als nächstes mittels der Zuordnungseinheit (1) durch Verdrehen der Scheibe (8) relativ zum Halter (7) die Zwischenwerte für den Bodymass-Index (BMI) und den prozentualen Gewichtsverlust ermittelt und den ermittelten Zwischenwerten mittels der Farbskalen (14) und (18) entsprechende Farben zugeordnet.

Alternativ zu der in den Abbildungen Fig. 1, 2 und 3 dargestellten Ausbildung der Zuordnungseinheit (1), bestehend aus wenigstens zwei relativ zueinander um eine gemeinsame Achse (6) verdrehbaren Bauteilen (7, 8), ist es selbstverständlich auch möglich, die Zuordnungseinheit (1) aus wenigstens zwei relativ zueinander verschiebbaren Bauteilen bestehend aufzubauen.

Im nächsten Schritt werden mittels des Informationsblockes (2) die weiteren drei Zwischenwerte für die Teilbereiche "Nahrungsaufnahme" (20), "Schwere der Erkrankung" (21) sowie "Alter" (22) ermittelt, wobei diesen Zwischenwerten über die farbig ausgestalteten Informationsfelder (23) entsprechende Farben zugeordnet werden.

Die den ermittelten Zwischenwerten entsprechenden Farben werden anschließend über die farbigen Tastenfelder (24) in die Auswerteeinheit (3) eingegeben, die mittels eines Auswertungsalgorithmus aus diesen Zwischenwerten den ernährungsspezifischen Endwert bestimmt und das Ergebnis in den Ergebnisfeldern (25) als optisch wahrnehmbares Farbsignal anzeigt.

Das Bestimmen des Endwertes in der Auswerteeinheit (3) mittels des Auswertungsalgorithmus geschieht wie folgt:

Zunächst wird jeder einen Zwischenwert verkörpernden Farbe ein Zahlenwert zugeordnet, nämlich:
der Farbe Grün der Wert 0;
der Farbe Gelb der Wert 1;
der Farbe Orange der Wert 2 und
der Farbe Rot der Wert 3.

In einem ersten Berechnungsschritt des Algorithmus werden die Zahlenwerte der Zwischenwerte für den "Body-mass-Index (BMI)", den "Gewichtsverlust" sowie für die "Nahrungsaufnahme" derart abgeglichen, dass nur der höchste Zahlenwert zur Bestimmung des Endwertes herangezogen wird und die anderen beiden Zwischenwerte unberücksichtigt bleiben.

In einem zweiten Berechnungsschritt des Algorithmus werden die Zahlenwerte der Zwischenwerte für das "Alter" und die "Schwere der Erkrankung" sowie der Zahlenwert des ersten Berechnungsschrittes addiert.

Aus der im zweiten Berechnungsschritt bestimmten Summe der Zahlenwerte wird mit folgender Maßgabe:
Endwert kleiner 3 = gelbes Signal und
Endwert größer/gleich 3 = rotes Signal
der gesuchte ernährungsspezifische Endwert bestimmt, wobei das Aufleuchten des gelben Ergebnisfeldes (25) als Endwert bedeutet, dass keine Ernährungsunterstützung erforderlich ist, und das Aufleuchten des roten Ergebnisfeldes (25) als Endwert bedeutet, dass eine Ernährungsunterstützung (möglichst täglich) erforderlich ist.

Mit Hilfe der voranstehend beschriebenen Vorrichtung ist es somit möglich, auf einfache Art und Weise schnell und ohne Laboranalysen wenigstens einen ernährungsspezifischen Wert zu bestimmen, der eine Aussage darüber gibt, ob der untersuchte Patient einer Ernährungsunterstützung bedarf. Die Ernährungsunterstützung kann dann dem Patienten enteral und/oder parenteral zugeführt werden.

### Bezugszahlen

- 1: Zuordnungseinheit
- 2: Informationsblock
- 3: Auswerteeinheit
- 4: Grundkörper
- 5: Falz
- 6: Achse
- 7: Halter
- 8: Scheibe
- 9: Gewichtsskala
- 10: Gewichtsskala
- 11: Ausnehmung
- 12: Pfeil
- 13: Markierung
- 14: Farbskala
- 15: Gewichtsskala
- 16: Größenskala
- 17: Ausnehmung
- 18: Farbskala
- 19: Pfeil
- 20: Teilbereich
- 21: Teilbereich
- 22: Teilbereich
- 23: Informationsfeld
- 24: Tastenfeld
- 25: Ergebnisfeld
- 26: Frageblock
- 27: kartenförmiges Material mit Aufklebern
- 28: Tasche

## Patentansprüche

1. Vorrichtung zur Bestimmung wenigstens eines ernährungsspezifischen Wertes zur Bestimmung des Ernährungsstatus eines Patienten mit einer Zuordnungseinheit zur Ermittlung wenigstens eines Zwischenwertes, wobei mittels der Zuordnungseinheit die Körpergröße und das Körpergewicht eines Patienten zur Ermittlung des Bodymass-Index (BMI) als ein Zwischenwert in Relation zueinander setzbar sind, und wobei mittels der Zuordnungseinheit zusätzlich das aktuelle Körpergewicht und das übliche Körpergewicht eines Patienten zur Ermittlung des prozentualen Gewichtsverlustes als ein weiterer Zwischenwert in Relation setzbar sind, einem Informationsblock zur Ermittlung wenigstens eines weiteren Zwischenwertes sowie mit einer Auswerteeinheit zur Bestimmung eines Endwertes aus dem wenigstens einen Zwischenwert der Zuordnungseinheit und dem wenigstens einen Zwischenwert des Informationsblockes,
**dadurch gekennzeichnet ,**
- **dass** die Zuordnungseinheit (1) mindestens zwei relativ zueinander verstellbare Bauteile (7, 8) zur Ermittlung des Bodymass-Index (BMI) aufweist,
- **dass** die Zuordnungseinheit (1) zur Ermittlung von zwei Zwischenwerten ausgebildet ist, wobei die Vorderseite zur Ermittlung des einen Zwischenwertes und die Rückseite zur Ermittlung des anderen Zwischenwertes ausgebildet ist,
- **dass** die Zuordnungseinheit (1) auf der Vorderseite eine Skala (14) mit den Zwischenwerten zugeordneten Farben, Symbolen oder Zahlen und auf der Rückseite eine Skala (18) mit den Zwischenwerten zugeordneten Farben,. Symbolen oder Zahlen aufweist,
- und **dass** die Auswerteeinheit Tastenfelder (24) aufweist, die den Zwischenwerten zugeordneten Farben, Symbolen oder Zahlen entsprechen.

2. Vorrichtung nach Anspruch 1, **dadurch gekennzeichnet, dass** mit der Zuordnungseinheit (1) wenigstens zwei patientenspezifische Werte zur Ermittlung wenigstens eines Zwischenwertes in Relation zueinander setzbar sind.

3. Vorrichtung nach mindestens einem der Ansprüche 1 oder 2, **dadurch gekennzeichnet, dass** die Zuordnungseinheit (1) aus wenigstens zwei relativ zueinander um eine gemeinsame Achse (6) verdrehbaren Bauteilen (7, 8) bestehend aufgebaut ist, wobei beide Bauteile (7, 8) mit wenigstens einer Skala (9, 10; 15, 16) versehen sind.

4. Vorrichtung nach mindestens einem der Ansprüche 1 bis 3, **dadurch gekennzeichnet, dass** die Zuordnungseinheit (1) aus wenigstens zwei relativ zueinander verschiebbaren Bauteilen bestehend aufgebaut ist, wobei beide Bauteile mit wenigstens einer Skala versehen sind.

5. Vorrichtung nach mindestens einem der Ansprüche 1 bis 4, **dadurch gekennzeichnet, dass** der Informationsblock (2) vorgegebene allgemeine Angaben zum Gesundheitsstatus enthält, die zur Ermittlung wenigstens eines weiteren Zwischenwertes mit den konkreten Angaben zum Gesundheitsstatus eines Patienten vergleichbar sind.

6. Vorrichtung nach Anspruch 5, **dadurch gekennzeichnet, dass** die im Informationsblock (2) enthaltenen, vorgegebenen "allgemeine" Angaben zum Gesundheitsstatus in die Teilbereiche Nahrungsaufnahme (20), Schwere der Erkrankung (21) und Alter (22) des Patienten unterteilbar sind, aus denen jeweils ein Zwischenwert ermittelbar ist.

7. Vorrichtung nach mindestens einem der Ansprüche 1 bis 6, **dadurch gekennzeichnet, dass** jedem ermittelten Zwischenwert über eine zugehörige Farbskala (14, 18, 23) eine individuelle Farbe zuordbar ist.

8. Vorrichtung nach mindestens einem der Ansprüche 1 bis 7, **dadurch gekennzeichnet, dass** in der Auswerteeinheit (3) aus den Zwischenwerten mittels einer elektronisch, elektrisch, elektromechanisch oder mechanisch arbeitenden Datenverarbeitungseinheit ein Endwert bestimmbar ist.

9. Vorrichtung nach Anspruch 8, **dadurch gekennzeichnet, dass** die Auswerteeinheit (3) in der Art eines elektronischen Taschenrechners ausgebildet ist.

10. Vorrichtung nach Anspruch 9, **dadurch gekennzeichnet, dass** in die Auswerteeinheit (3) die den Zwischenwerten entsprechenden Farben als Eingangsdaten eingebbar sind.

11. Vorrichtung nach Anspruch 10, **dadurch gekennzeichnet, dass** die Datenverarbeitungseinheit der Auswerteeinheit (3) mittels eines Auswertungsalgorithmus arbeitet, der jeder unterschiedlichen Farbe eines Zwischenwertes einen unterschiedlichen Zahlenwert zuordnet.

12. Vorrichtung nach mindestens einem der Ansprüche 1 bis 11, **dadurch gekennzeichnet, dass** der Endwert als optisches, insbesondere farbiges Signal darstellbar ist.

13. Vorrichtung nach mindestens einem der Ansprüche 1 bis 12, **dadurch gekennzeichnet, dass** die Zuordnungseinheit (1), der Informationsblock (2) sowie die Auswerteeinheit (3) auf einem faltbaren Grundkörper (4) angeordnet sind.

14. Vorrichtung nach Anspruch 13, **gekennzeichnet durch** einen zusätzlichen, auf dem Grundkörper (4) angeordneten Frageblock (26).

15. Vorrichtung nach Anspruch 13 oder 14, **dadurch gekennzeichnet, dass** am Grundkörper (4) wenigstens eine Tasche (28) ausgebildet ist.

## Claims

1. A device for determining at least one nutrition-specific value for determining the nutritional status of a patient, with a reference unit for establishing at least one intermediate value, wherein the height and body weight of a patient can be placed in relation to one another by means of the reference unit in order to establish the body mass index (BMI) as an intermediate value, and wherein the current body weight and the usual body weight of a patient can additionally be placed in relation to one another by means of the reference unit in order to establish the percentage weight loss as a further intermediate value, with an information block for establishing at least one further intermediate value, and with an evaluation unit for determining a final value from the at least one intermediate value from the reference unit and the at least one intermediate value from the information block, **characterised in that**
- the reference unit (1) has at least two parts (7, 8), which are displaceable relative to one another, for establishing the body mass index (BMI),
- **in that** the reference unit (1) is formed for establishing two intermediate values, wherein the front is formed for establishing the one intermediate value and the back is formed for establishing the other intermediate value,
- **in that** the reference unit (1) has, on the front, a scale (14) with colours, symbols or numbers assigned to the intermediate values and, on the back, a scale (18) with colours, symbols or numbers assigned to the intermediate values,
- and **in that** the evaluation unit has banks of keys (24) which correspond to the colours, symbols or numbers assigned to the intermediate values.

2. A device according to claim 1, **characterised in that** at least two patient-specific values for establishing at least one intermediate value can be placed in relation to one another by means of the reference unit (1).

3. A device according to at least one of claims 1 and 2, **characterised in that** the reference unit (1) is constructed from at least two parts (7, 8) which are rotatable relative to one another about a common axis (6), wherein both parts (7, 8) are provided with at least one scale (9, 10; 15, 16).

4. A device according to at least one of claims 1 to 3, **characterised in that** the reference unit (1) is constructed from at least two parts which are displaceable relative to one another, wherein both parts are provided with at least one scale.

5. A device according to at least one of claims 1 to 4, **characterised in that** the information block (2) contains specified general health-status data which can be compared with the actual health-status data of a patient in order to establish at least one further intermediate value.

6. A device according to claim 5, **characterised in that** the specified "general" health-status data contained in the information block (2) can be divided into the categories of food intake (20), severity of the illness (21) and age (22) of the patient, from each of which an intermediate value can be established.

7. A device according to at least one of claims 1 to 6, **characterised in that** an individual colour can be assigned to each established intermediate value by means of an associated colour scale (14, 18, 23).

8. A device according to at least one of claims 1 to 7, **characterised in that** a final value is determinable in the evaluation unit (3) from the intermediate values by means of an electronically, electrically, electromechanically or mechanically operating data processing unit.

9. A device according to claim 8, **characterised in that** the evaluation unit (3) is formed in the manner of an electronic pocket calculator.

10. A device according to claim 9, **characterised in that** the colours corresponding to the intermediate values can be input into the evaluation unit (3) as input data.

11. A device according to claim 10, **characterised in that** the data processing unit of the evaluation unit (3) operates by means of an evaluation algorithm which assigns a different numerical value to each different colour of an intermediate value.

12. A device according to at least one of claims 1 to 11, **characterised in that** the final value can be displayed as an optical signal, especially a colour signal.

13. A device according to at least one of claims 1 to 12, **characterised in that** the reference unit (1), the information block (2) and the evaluation unit (3) are arranged on a folding main body (4).

14. A device according to claim 13, **characterised by** an additional question block (26) arranged on the main body (4).

15. A device according to claim 13 or 14, **characterised in that** at least one pocket (28) is formed on the main body (4).

## Revendications

1. Dispositif pour déterminer au moins une valeur nutritionnelle pour définir l'état de nutrition d'un patient avec une unité d'affectation pour déterminer au moins une valeur intermédiaire, moyennant quoi la taille et le poids du corps d'un patient peuvent être définis l'un par rapport à l'autre au moyen de l'unité d'affectation pour déterminer l'indice de masse corporelle (IMC) comme valeur intermédiaire, et où le poids actuel du corps et le poids du corps normal d'un patient peuvent également être définis l'un par rapport à l'autre au moyen de l'unité d'affectation pour déterminer le pourcentage de perte de poids comme autre valeur intermédiaire, avec un bloc d'informations pour déterminer au moins une autre valeur intermédiaire ainsi qu'avec une unité d'analyse pour déterminer une valeur finale à partir d'au moins une valeur intermédiaire de l'unité d'affectation et d'au moins une valeur intermédiaire du bloc d'informations,
**caractérisé en ce que**
- l'unité d'affectation (1) comporte au moins deux éléments (7, 8) réglables relativement l'un par rapport à l'autre pour déterminer l'indice de masse corporelle (IMC),
- l'unité d'affectation (1) est conçue pour déterminer deux valeurs intermédiaires, et la face avant est conçue pour définir une des valeurs intermédiaires et la face arrière est conçue pour définir une autre des valeurs intermédiaires,
- l'unité d'affectation (1) comporte, sur sa face avant, une échelle (14) avec des couleurs, symboles ou chiffres affectés aux valeurs intermédiaires et, sur sa face arrière, une échelle (18) avec des couleurs, des symboles ou des chiffres affectés aux valeurs intermédiaires,
- et **en ce que** l'unité d'analyse comporte des touches (24) qui correspondent aux couleurs, symboles ou chiffres affectés aux valeurs intermédiaires.

2. Dispositif selon la revendication 1, **caractérisé en ce qu'**au moins deux valeurs propres au patient peuvent être définies l'une par rapport à l'autre à l'aide de l'unité d'affectation (1) pour déterminer au moins une valeur intermédiaire.

3. Dispositif selon au moins l'une quelconque des revendications 1 et 2, **caractérisé en ce que** l'unité d'affectation (1) est composée d'au moins deux éléments (7, 8) pivotant l'un par rapport à l'autre autour d'un axe commun (6), et les deux éléments (7, 8) sont pourvus d'au moins une échelle (9, 10 ; 15, 16).

4. Dispositif selon au moins l'une quelconque des revendications 1 à 3, **caractérisé en ce que** l'unité d'affectation (1) est composée d'au moins deux éléments mutuellement mobiles, et les deux éléments sont pourvus d'au moins une échelle.

5. Dispositif selon au moins l'une quelconque des revendications 1 à 4, **caractérisé en ce que** le bloc d'informations (2) contient des données générales prédéfinies sur l'état de santé, lesquelles peuvent être comparées pour déterminer au moins une autre valeur intermédiaire avec les données concrètes sur l'état de santé d'un patient.

6. Dispositif selon la revendication 5, **caractérisé en ce que** les données "générales" prédéfinies contenues dans le bloc d'informations (2) relatives à l'état de santé peuvent être divisées dans les sous-domaines Alimentation (20), Gravité de la maladie (21) et Âge (22) du patient, à partir desquels il est possible de déterminer une valeur intermédiaire.

7. Dispositif selon au moins l'une quelconque des revendications 1 à 6, **caractérisé en ce qu'**une couleur individuelle peut être affectée à chaque valeur intermédiaire déterminée sur une échelle de couleurs correspondante (14, 18, 23).

8. Dispositif selon au moins l'une quelconque des revendications 1 à 7, **caractérisé en ce qu'**une valeur finale peut être déterminée dans l'unité d'analyse (3) à partir des valeurs intermédiaires au moyen d'une unité de traitement des données électronique, électrique électromécanique ou mécanique.

9. Dispositif selon la revendication 8, **caractérisé en ce que** l'unité d'analyse (3) est conçue comme une calculatrice de poche électronique.

10. Dispositif selon la revendication 9, **caractérisé en ce que** les couleurs correspondant aux valeurs intermédiaires dans l'unité d'analyse (3) peuvent être introduites comme données d'entrée.

11. Dispositif selon la revendication 10, **caractérisé en ce que** l'unité de traitement des données de l'unité d'analyse (3) travaille à l'aide d'un algorithme d'analyse, lequel affecte, à chaque couleur différente d'une valeur intermédiaire, une valeur chiffrée différente.

12. Dispositif selon au moins l'une quelconque des revendications 1 à 11, **caractérisé en ce que** la valeur finale peut être représentée sous forme de signal optique, plus particulièrement de signal de couleur.

13. Dispositif selon au moins l'une quelconque des revendications 1 à 12, **caractérisé en ce que** l'unité d'affectation (1), le bloc d'informations (2) ainsi que l'unité d'analyse (3) sont placés sur un corps de base (4) pliable.

14. Dispositif selon la revendication 13, **caractérisé par** un bloc répondeur (26) supplémentaire placé sur le corps de base (4).

15. Dispositif selon la revendication 13 ou 14, **caractérisé en ce qu'**au moins une poche (28) est placée sur le corps de base (4).
